(19) Europäisches Patentamt
European Patent Office
Office européen des brevets

(11) Publication number: **0 024 112 B1**

(12) **EUROPEAN PATENT SPECIFICATION**

(45) Date of publication of patent specification: **01.02.84**

(51) Int. Cl.$^3$: **C 07 D 207/02, G 01 N 33/72**

(21) Application number: **80302364.7**

(22) Date of filing: **11.07.80**

(54) **Method for the separation and isolation of conjugated and unconjugated bilirubin, isolated conjugated bilirubin, and reference composition containing same.**

(30) Priority: **11.07.79 US 56585**
**10.12.79 US 101663**

(43) Date of publication of application:
**25.02.81 Bulletin 81/8**

(45) Publication of the grant of the patent:
**01.02.84 Bulletin 84/5**

(84) Designated Contracting States:
**BE CH DE FR GB IT LI NL**

(56) References cited:
**EP - A - 0 008 233**
**US - A - 4 069 017**

**CLINICAL CHEMISTRY, Vol. 25, No. 6, 1979 T.W. WU et al. "Human Conjugated Bilirubin-Isolation, Biosynthesis, and Molecular Characterizations by Direct Spectroscopic Analyses", page 1137**

**The file contains technical information submitted after the application was filed and not included in this specification**

(73) Proprietor: **EASTMAN KODAK COMPANY**
**343 State Street**
**Rochester New York 14650 (US)**

(72) Inventor: **Wu, Tai-Wing**
**Kodak Park**
**Rochester, New York (US)**

(74) Representative: **Pepper, John Herbert et al,**
**KODAK LIMITED Patent Department P.O. Box 114**
**190 High Holborn**
**London WC1V 7EA (GB)**

(56) References cited:
**Chemical Abstracts vol. 90, no. 15, 9 April 1979 Columbus, Ohio, USA C.K. LIM "The separation of conjugated and unconjugated bilirubin in bile by high-performance liquid chromatography", page 271, column 1, abstract no. 117341m**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 024 112 B1**

Courier Press, Leamington Spa, England.

# Method for the separation and isolation of conjugated and unconjugated bilirubin, isolated conjugated bilirubin, and reference composition containing same

It has been generally assumed by clinical chemists that conjugated bilirubin ($B_c$) in humans is chiefly a diglucuronide and that $B_c$ exists in the same molecular form in a variety of different body fluids which contain this metabolic product. Owing to the unstable nature of $B_c$, however, its isolation and specific molecular characterization have been extremely difficult.

Definitive molecular characterization of $B_c$ has, to the knowledge of this inventor, been carried out for the first time by this inventor and his co-workers. See paper entitled "Human Conjugated Bilirubin — Isolation, Biosynthesis, and Molecular Characterization By Direct Spectroscopic Analyses" presented by T. W. Wu et al at the American Association for Clinical Chemistry 31st Annual Meeting in New Orleans, Louisiana, July 15—20, 1979. An abstract of this paper appears in *Clinical Chemistry*, Vol. 25, No. 6, p. 1137 (June, 1979). To determine the molecular structure of $B_c$, $B_c$ was isolated by the bile extraction and isolation procedure reported by Lucassen, J., doctoral thesis, University of Utrecht, Netherlands (1961). Bile was chosen for the separation and isolation of $B_c$ because of the high concentration of $B_c$ which exists in bile. The principal molecular species of $B_c$ isolated from bile by the Lucassen procedure has now been found not to be a diglucuronide as many have previously speculated, but rather a diester having a molecular weight of 918.2 and containing one molecule of glucuronic acid and one of glucuronolactone as shown in Formula I:

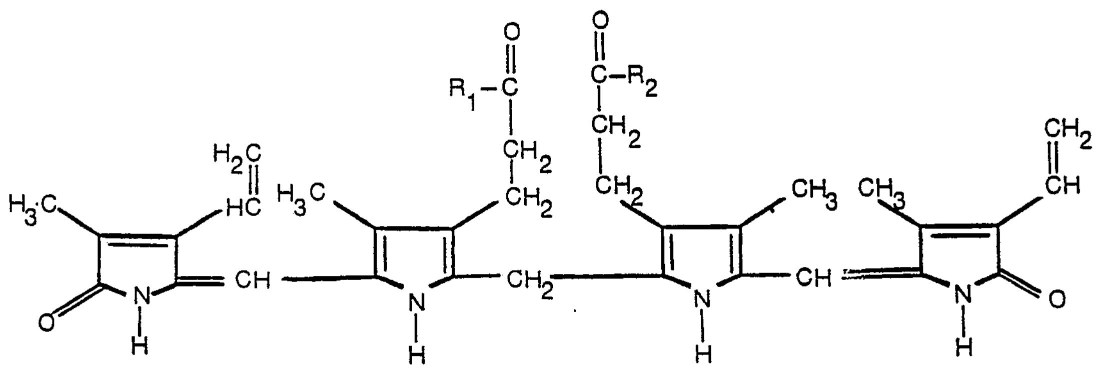

where $R_1$ = glucuronic acid and $R_2$ = glucuronolactone or $R_1$ = glucuronolactone and $R_2$ = glucuronic acid.

The presence and concentration of $B_c$ in body fluids is of diagnostic significance, particularly in dealing with the treatment of certain jaundice conditions, e.g., in obstructive jaundice the small amount of $B_c$ normally present in adult human serum becomes elevated to form a larger proportion of the total bilirubin content. The total bilirubin content consists of the sum of $B_c$ and the more familiar, predominant form of bilirubin, referred to as unconjugated bilirubin ($B_u$).

Colorimetric and fluorimetric assay methods for bilirubin using interactive mordants are described in U.S. Patent 4,017,069 and *Research Disclosure* Vol. 175, November 1978, Item 17554.

In the course of developing the foregoing assay methods for bilirubin, it became apparent that improved methods for separating and isolating $B_c$ and/or $B_u$ from aqueous liquids, particularly those containing mixtures of both $B_c$ and $B_u$, were necessary owing to the need for having $B_c$ and $B_u$ standards effective to calibrate assays for $B_c$ and $B_u$. At present, $B_u$ standards that can be purchased typically come from nonhuman bile sources, e.g., cow or ox, while $B_c$ standards are not available from either human or nonhuman animal sources. Various artificial standards have been used for bilirubin, such as ferric thiocyanate, cobalt sulfate, and potassium permanganate. However, as reported by Winkelman, J. et al, *Clinical Chemistry — Principles and Technics,* R. J. Henry, D. C. Cannon, and J. W. Winkelman, Eds., Harper & Row Publishers, New York, 2nd Ed., 1974, pp 1038—1070, these artificial standards do not work well in spectrophotometric assays, because their absorption curves are not the same as that of bilirubin.

Although one could attempt to obtain a $B_c$ standard by use of the above-referenced Lucassen procedure, this procedure is difficult to perform. In addition, the Lucassen procedure provides little or no stabilization of $B_c$ during the isolation and extraction process, and therefore the $B_c$ has a pronounced tendency to degrade during the process. Moreover, the Lucassen procedure, although useful on bile which contains relatively high concentrations of $B_c$ and low concentrations of protein to which bilirubin readily binds, has never, to the knowledge of the inventor, been successfully applied to other aqueous biological liquids. For example, the Lucassen procedure has been found unsuccessful when applied to serum which contains relatively low amounts of $B_c$ and high concentrations of protein. Accordingly, a new method for separating and isolating $B_c$ and/or $B_u$ from bile as well as other aqueous liquids such as serum would be highly desirable.

Once separated and isolated, $B_c$ and/or $B_u$ can be employed for numerous clinical purposes, such as in the manufacture and preparation of various standards, i.e., reference compositions, such as calibrators and controls, used in the assay of bilirubin contained in biological fluids such as serum, urine, cerebrospinal fluid and bile.

The present invention provides a new method for the separation of conjugated and unconjugated bilirubin from aqueous liquids containing bilirubin, especially mixtures of $B_c$ and $B_u$.

According to the present invention there is provided a method for the separation and isolation of conjugated bilirubin ($B_c$) and unconjugated bilirubin ($B_u$) from an aqueous liquid containing $B_c$ and/or $B_u$, said method characterized by:

a) bringing the liquid and an interactive mordant for $B_c$ and $B_u$ into contact;

b) separating the $B_c$ and/or $B_u$ mordanted in step (a) from the liquid; and

c) treating the separated mordanted $B_c$ and/or $B_u$ of step (b) in an aqueous medium with a chaotropic agent which is soluble in the medium to release at least a portion of the $B_c$ and/or $B_u$ from the mordant.

By the term "interactive mordant" we mean that the $B_c$ and $B_u$ enter into a chemical reaction opposed to mere physical absorption.

In a preferred embodiment of the present method, the chaotropic agent employed in step (c) is selected from ionizable salts; xanthine; alkylated xanthines, e.g., caffeine; non-ionic surfactants, e.g., alkarylpolyethers; urea or mixtures of the foregoing materials. Especially preferred chaotropic agents include ionizable salts; mixtures of sodium benzoate and an alkylated xanthine; and non-ionic surfactants.

In an especially preferred embodiment, $B_c$ and $B_u$ are also selectively separated from one another. This is achieved during or after step (c) by selectively dissolving either the released $B_c$ or the released $B_u$. For example, in one embodiment, step (c) of the present method is carried out at a pH at which the $B_c$ is soluble in the aqueous medium while the $B_u$ forms an insoluble solid phase. Accordingly, this embodiment effectively isolates $B_c$ from $B_u$. In another aspect of this embodiment, the selective separation of $B_c$ from $B_u$ can be achieved or further enhanced by addition of a water-miscible organic solvent following step (b). For example, propanol and other alkyl alcohols partially dissolve $B_c$ while $B_u$ is substantially insoluble in these alcohols. In yet another aspect of this embodiment, the released $B_u$ is separated from the released $B_c$ by extraction in a water-immiscible organic solvent to dissolve $B_u$, e.g., chloroform, dichloromethane, or a mixture thereof.

A further embodiment of the invention provides isolated $B_c$ having a purity in excess of 75% by weight, preferably in excess of 85% by weight as determined by quantitative NMR (nuclear magnetic resonance) and gravimetric analysis.

A reference composition including the isolated $B_c$ for assay of bilirubin contained in an aqueous liquid is also provided. This reference composition includes a wet or dry matrix, and the isolated $B_c$. In the case of a reference composition containing a dry matrix, upon addition of an aqueous liquid, the resultant aqueous composition represents a useful reference fluid for calibrating clinical elements and reagents intended for assay of bilirubin in biological liquids, particularly quantitative assays of $B_c$ and total bilirubin, i.e., the sum of $B_c$ and $B_u$ contained in an aqueous liquid.

The present invention provides a new method for the separation and isolation of conjugated bilirubin ($B_c$) and/or unconjugated bilirubin ($B_u$) from aqueous liquids containing mixtures of $B_u$ and $B_c$. The method, of course, is also applicable to aqueous liquids in which the entire bilirubin content is composed solely of either $B_c$ or $B_u$. Because of the difficulty in distinguishing between the $B_c$ and $B_u$ components present in aqueous liquids containing mixtures of these components, and because $B_c$ is usually present in much lower concentration than $B_u$ and is also highly unstable and otherwise difficult to isolate, the method of the invention is particularly useful in the isolation of $B_c$ from aqueous liquids containing mixtures of $B_c$ and $B_u$.

The method of the invention can advantageously be employed to isolate $B_c$ and/or $B_u$ from a variety of aqueous liquids. For example, the method can be applied to aqueous biological liquids such as bile, serum, cerebrospinal fluid, and urine, derived from both human and nonhuman animal sources. In particular, the method can be applied to aqueous liquids that contain relatively low amounts of $B_c$ and relatively large amounts of protein to which bilirubin is known to bind. A further advantage of the present method is that the interactive mordants used appear to aid in stabilizing $B_c$ during the separation and isolation steps so that there is less tendency for the $B_c$ to degrade.

The environmental conditions, including the temperature as well as the atmosphere under which the present method is carried out, can vary considerably. However, owing to the susceptibility of bilirubin to degradation in oxidizing atmospheres and the general instability of bilirubin, particularly $B_c$, the method is preferably carried out in a non-oxidizing atmosphere, such as under nitrogen or an inert gas such as argon. Likewise, the temperature is preferably maintained within a range effective to avoid degradation of bilirubin, for example, within a range of from 0 to 60°C, preferably from 0 to 10°C. By using the foregoing preferred conditions, one can optimize the amounts of $B_c$ and $B_u$ isolated.

To optimize further the amounts of $B_c$ and $B_u$ isolated, the present method is preferably carried out in the dark or under yellow safe-light conditions to avoid light-induced degradation of bilirubin.

In step (a) of the present method, the liquid containing a quantity of $B_c$ and/or $B_u$ to be isolated is

contacted with an interactive mordant to mordant both $B_c$ and $B_u$. This can be carried out by mixing together an aqueous liquid containing the mordant dissolved or suspended therein and the aqueous bilirubin-containing liquid. Alternatively, the mordant can be mixed directly in the aqueous bilirubin-containing liquid as a dry powder; or the aqueous bilirubin-containing liquid can be applied to an essentially dry zone, e.g., a layer, comprising the mordant, for example, the dry test element described in U.S. Patent 4,069,017. In still another embodiment, the interactive mordant can be packed in a column and the aqueous liquid containing the bilirubin can be passed through the column to contact the mordant.

The quantity of mordant used in this first step can vary widely depending upon the particular mordant selected, its binding capacity for $B_c$ and $B_u$, and the particular means whereby this step is carried out, e.g., whether the mordant is contained in an aqueous liquid at the time of contact, is present as a dry zone of a dry test element, or is packed in a column. To optimize the amount of $B_c$ or $B_u$ isolated by the present invention, one preferably employs in step (a) of the method an amount of mordant in excess of the maximum amount of $B_c$ and $B_u$ thought to be present in the liquid to ensure that as much bilirubin as possible is mordanted. Where the mordant is added as a dry powder to the aqueous bilirubin-containing liquid or where the mordant is first dissolved or suspended in an aqueous liquid and then added to the aqueous bilirubin-containing liquid, a useful amount of the preferred copolymer mordants described in greater detail hereinafter, is typically that which is sufficient to provide a final amount of mordant in the bilirubin-containing liquid within the range of from 0.007 to 10 g% based on the total weight of mordant and aqueous liquid.

A useful test for determining the amount of a particular interactive mordant to be contacted with a particular bilirubin-containing aqueous liquid can readily be performed by monitoring the absorption maxima of the mordanted bilirubin. That is, mordanted bilirubin exhibits enhanced absorptivity because of the interaction of the mordant and bilirubin. This is described in U.S. Patent 4,016,017. The respective absorption maxima of mordanted $B_c$ and $B_u$ can vary somewhat depending upon the particular interactive mordant, but an absorption maximum for mordanted $B_c$ usually occurs at 420—430 $\pm$20 nm and for mordanted $B_u$ at 460 nm $\pm$20 nm. Thus, by monitoring the absorption maxima of mordanted $B_u$ and $B_c$ as the aqueous liquid sample is contacted with increasing amounts of the mordant, the addition of the mordant can be terminated when the absorption maxima of mordanted $B_c$ and mordanted $B_u$ stop increasing.

The length of time for contacting the bilirubin-containing liquid and the interactive mordant can vary, depending upon the concentration of bilirubin in the liquid, the amount of mordant present, the binding capability of the mordant, and the degree of intermolecular contact between the mordant and the bilirubin. A contact time within the range of from 10 seconds to 10 minutes is usually effective, although shorter or longer contact times can also be used depending upon the particular circumstances.

The pH at which the bilirubin-containing liquid and mordant are contacted can vary over a relatively wide range. For example, the pH can range from 2 to 10, $B_u$ being soluble from pH 7.4 to 10 and $B_c$ being soluble from pH 2 to 10. Where the aqueous liquid from which bilirubin is to be isolated is serum, the pH of the first step can be carried out essentially at the pH of the serum. Normal human serum has a pH of 7.35 $\pm$.05.

The step of contacting the aqueous, bilirubin-containing liquid and the mordant can be carried out under conditions under which the mordant is partially solubilized or readily suspended in aqueous liquid to ensure good interaction between the mordant and bilirubin. In some cases, therefore, depending upon the particular mordant selected and its solubility in water, various organic liquid solubilizing agents for the mordant can be added that are miscible with the aqueous bilirubin-containing liquid, such as lower alkyl alcohols, lower alkyl ethers, and lower alkyl ketones, containing 1 to 3 carbon atoms in the alkyl group, for example, methanol, ethanol, and acetone, and cyclic ethers, such as tetrahydrofuran.

Having mordanted some, and preferably most of all, of the bilirubin present in the bilirubin-containing liquid, the mordanted bilirubin is separated from the aqueous liquid in step (b) of the present method. Where step (a) of the method is carried out in an aqueous liquid medium, step (b) can readily be carried out by centrifuging because of the low solubility of the mordanted bilirubin in aqueous liquids. However, other separation techniques, including various filtration methods, are also useful. Separation is preferably carried out at a temperature within the range of from 0°C to 10°C. Bilirubin is believed to be more stable at this reduced temperature and the solubility of the mordanted bilirubin may also be decreased, thereby facilitating the separation of the mordanted bilirubin from the aqueous liquid phase. Separation by centrifuging can conveniently be carried out at 15000 x g for 5 to 15 minutes. However, other centrifuging conditions can also be used. The aforementioned conditions are merely representative and are not to be regarded as critical.

Where step (a) of the present method is carried out by use of an essentially dry zone containing the mordant, such as by use of the dry test element described in U.S. 4,069,017, the separation of the mordanted bilirubin from the aqueous liquid originally containing the bilirubin can readily be carried out in accord with step (b) of the present method as follows: The aqueous liquid is allowed to penetrate into the mordant-containing zone of the element upon application of the liquid to the element in step

(a), the bilirubin in the liquid binds to the mordant, and the mordanted bilirubin can then be isolated from the dry mordant-containing zone of the element upon evaporation of the aqueous liquid.

Having separated the mordanted bilirubin in step (b) of the present method, the $B_c$ and $B_u$ can then be released from the interactive mordant in step (c). To do this, the mordanted $B_c$ and $B_u$ are treated under appropriate pH conditions in an aqueous medium with a chaotropic agent at least slightly soluble in the aqueous medium. This treatment releases the bilirubin (both $B_c$ and $B_u$) from the mordant to which it is bound.

The chaotropic agent used in step (c) may be a very water-soluble or at least slightly water-soluble material that disrupts the bond of the mordant with $B_u$ and/or $B_c$. This bond is believed to occur through both ionic bonds and hydrophobic site bonds and a chaotropic agent effective to disrupt at least one of these bonding mechanisms is useful for releasing at least a portion of the $B_u$ and/or $B_c$ bound to the mordant.

One preferred chaotropic agent is a salt ionizable in the aqueous medium. The usefulness of the salt in releasing the $B_c$ and/or $B_u$ from the mordant is believed to be brought about by the reduction of the ionic binding capacity between the mordant and the bilirubin molecules in the presence of the soluble ions derived from the salt. A variety of salts can be used for this purpose. The salt should be at least slightly soluble in the aqueous medium and contain groups ionizable in the medium. Salts of strong acids and strong bases are preferred because of their ability to undergo essentially complete ionization and provide a large number of ions to the aqueous medium. In addition, because of this ability to undergo essentially complete ionization, the salts of strong acids and strong bases can readily be removed from the aqueous medium following release of $B_c$ and $B_u$, which is often desirable. Sodium chloride works especially well as the ionizable salt. Other monovalent alkali metal and alkaline earth metal salts are also preferred. However, a variety of other soluble, ionizable salts including polyvalent salts, ionic surfactants, and buffers, e.g., tris(hydroxymethyl)aminomethane hydrochloride and sodium citrate can be used. A partial listing of representative ionizable salts considered useful in the invention includes, in addition to sodium chloride, the following: potassium chloride, ammonium sulfate, cesium chloride, guanidinium hydrochloride and sodium benzoate.

The amount of salt employed to release the bound bilirubin will depend, in large part, on the particular salt, its solubility, and its degree of ionization in the aqueous medium selected. When employing salts of strong acids and strong bases, amounts of the salt effective to bring its molar concentration in the aqueous medium within the range of from 0.1 M to 2 M have been found useful. However, for other types of salts, concentrations outside the aforementioned molarity values can also be used.

Non-ionic surfactants at least slightly soluble in the aqueous medium of step (c) also represent a preferred class of chaotropic agent effective to release $B_c$ or $B_u$ from the mordant. The release of $B_u$ or $B_c$ from the interactive mordant in the presence of these non-ionic surfactants is thought to occur as the non-ionic surfactant interferes with and thereby disrupts the hydrophobic bonding sites on the mordant for the $B_c$ or $B_u$. A partial listing of representative non-ionic surfactants can be found in McCutcheon's *Detergents and Emulsifiers,* 1974 North American Edition by the Allured Publishing Corporation. Specific, useful non-ionic surfactants include alkarylpolyethers, such as alkylphenoxypoly-ethoxyethanols having from 1 to 9 carbon atoms in the alkyl group and 5 to 40 ethoxy groups. These alkarylpolyethers are, for example, available from the Rohm and Haas Company under the trade names Triton X—100, Triton X—102, Triton X—305 and Triton X—405. Other specific useful non-ionic surfactants are the polyoxyethylene sorbitan fatty acid esters such as polyoxyethylene sorbitan monolaurate, sold under the tradename of Tween 20 by ICI America, Inc., Atlas Chemicals Division. The amount of the non-ionic surfactant useful in the present invention can vary, but an amount of non-ionic surfactant effective to bring the concentration of the surfactant in the aqueous medium to a value within 0.1 to 10 g% has been found useful.

Other useful chaotropic agents include xanthine and xanthine homologs and derivatives, especially alkylated xanthines. Preferred alkylated xanthines include caffeine (1,3,7-trimethylxanthine), theobromine (3,7-dimethylxanthine), and theophylline (1,3-dimethylxanthine). One especially useful chaotropic agent comprises a mixture of xanthine and sodium benzoate, for example, a mixture of sodium benzoate and caffeine. The ratio of caffeine to sodium benzoate in the mixture can be varied. Typically, molar ratios of caffeine to sodium benzoate in the range of from 1:2 to 1:10 are considered useful. Yet another useful chaotropic agent is urea.

As will be appreciated from the foregoing discussion, a variety of different chaotropic agents are useful in the present invention and the invention is not limited to a particular type or class of these agents. Furthermore, although useful chaotropic agents should be at least slightly soluble in an aqueous medium, good or even moderate solubility is not required. Xanthines, for example, are only slightly soluble in water. However, best results are generally obtained with chaotropic agents of moderate to excellent solubility in the aqueous medium. For purposes of the present specification, the phrase "slightly soluble" refers to a material having a solubility in water equal to or greater than that of xanthine (also known as 2,6-purinedione).

Following completion of step (c), the $B_c$ and $B_u$ will have been effectively separated from the other components of the original aqueous liquid and from the mordant. If desired, and in accord with an

especially preferred embodiment of the present method, the $B_c$ and $B_u$ can also be effectively separated from each other. This can be achieved during or after step (c) of the method by selectively solubilizing $B_c$ or $B_u$. For example, in one embodiment, the mordanted $B_c$ and $B_u$ can be treated with a chaotropic agent in step (c) at a pH which selectively solubilizes $B_c$ in an aqueous liquid phase supernatant while $B_u$ forms an insoluble solid phase. In this embodiment, which makes use of the aforementioned pH solubility characteristics of $B_c$ and $B_u$, step (c) can be conducted at a pH less than 7.0, preferably 6.5 or lower, at which $B_c$ is soluble and $B_u$ is insoluble. In addition to the adjustment of pH conditions, a water-miscible organic solvent can be added following step (b), for example, during or after step (c), to separate or enhance separation of $B_c$ from $B_u$. For example, $B_c$ is soluble in propanol and propanol-water mixtures as well as in other water-miscible organic solvents such as alkyl alcohols having 1 to 10 carbon atoms in the alkyl group thereof and mixtures of these solvents with water. $B_u$, on the other hand, remains substantially insoluble in aqueous alkyl alcohol mixtures, especially if the pH is maintained below 7.0.

Separation and isolation of $B_u$ and $B_c$ from one another can also be achieved by organic solvent extraction of $B_u$ from $B_c$. That is, $B_u$ is more soluble than $B_c$ in water-immiscible organic solvents, such as chloroform and dichloromethane. Thus, by extracting the released $B_u$ and $B_c$ from step (c) in such water-immiscible organic solvents, one can preferentially remove $B_u$ from a mixture of $B_u$ and $B_c$.

In that preferred embodiment of the present method wherein $B_c$ is selectively isolated from $B_u$ during or following step (c) by appropriate adjustment of pH, the $B_c$ is dissolved in the aqueous liquid supernatant, and the $B_u$ is retained in the solid phase. The $B_c$-containing supernatant can readily be isolated from the $B_u$-containing solid phase by ordinary means, e.g., by centrifuging, decanting or filtering. Thereafter, if desired, the liquid can be partially or wholly removed from the supernatant such as by lyophilization (freeze-drying) to yield either a more concentrated aqueous solution of $B_c$ or a finely-divided powder containing $B_c$ together with the chaotropic agent and other impurities. Preferably, the liquid of the $B_c$-containing supernatant is only partially removed, and the resultant concentrated aqueous solution of $B_c$ is further treated to remove the chaotropic agent, thereby further purifying the $B_c$. For example, removal of a soluble, ionizable salt can be carried out by any one of a number of conventional separation techniques including, but not limited to, ultrafiltration, dialysis, and chromatographic techniques such as thin-layer chromatography and column chromatography. Column chromatography has been found especially useful, particularly molecular sieve column chromatography employing gel beads, such as LH—20 gel beads (LH—20 is a tradename of certain gel beads available from Pharmacia, Uppsala, Sweden), as the stationary phase and a lower alkyl alcohol-water mixture as the eluting medium.

Following these optional purification steps, aqueous media containing concentrated $B_c$, $B_u$, or mixtures of $B_c$ and $B_u$ can be stored, preferably in the dark or under yellow safe-light conditions, at low temperatures such as —75°C to 10°C under a vacuum or other non-oxidizing atmosphere to prevent degradation of the $B_c$. Preferably, the purified aqueous media containing concentrated $B_c$, $B_u$, or mixtures of $B_c$ and $B_u$ are freeze-dried to powder form for ease of storage. When needed, the dry powder can readily be reconstituted by addition of aqueous liquid.

To improve and optimize the yield of $B_c$ separated and isolated in accord with that embodiment of the present method wherein step (c) is carried out at a pH effective to solubilize $B_c$ while $B_u$ remains insoluble, one can repeat step (c). That is, after treating the mordanted bilirubin in the presence of an aqueous medium at a pH of less than 7.0 with a chaotropic agent to release $B_c$ and $B_u$ and solubilize $B_c$ in the aqueous liquid supernatant, one can separate the aqueous supernatant from the solid phase, retain the supernatant, and then subject the solid phase to one or more additional treatments at a pH less than 7.0 to remove any additional $B_c$ that may be present in the solid phase. Thereafter, each of the aqueous phase supernatants containing $B_c$ can be collected and combined to form a single solution of $B_c$.

In a further embodiment of the present method wherein $B_c$ is separated from $B_u$, the treatment of the mordanted bilirubin with the chaotropic agent in step (c) is carried out not only at a pH of less than 7.0 but also in the presence of an aqueous medium containing a water-miscible organic solvent that is volatile. The presence of this volatile solvent is particularly useful because, among other reasons, it expedites any subsequent liquid removal step that may be carried out to further concentrate and purify $B_c$. Typical of such volatile, water-miscible organic solvents are the alkyl alcohols having 1 to 10 carbon atoms in the alkyl group, mentioned above. When such volatile solvents are employed, care should be taken to avoid those solvents which will also solubilize the $B_u$. In this regard, the alcohols, octanol and propanol are considered especially effective.

Other optional additives such as antioxidants, for example, sodium thiosulfite or ascorbic acid, can be employed during the present method to aid in preventing or retarding the oxidation or other forms of chemical or physical degradation of bilirubin, especially $B_c$.

A further embodiment of the invention provides reference compositions, containing isolated $B_c$, useful as calibrators and controls for assays of aqueous liquids containing $B_c$. A major advantage of these reference compositions is that the isolated conjugated bilirubin contained therein is not only of high purity, i.e., in excess of 75%, preferably 85—90%, by weight or higher, but also is substantially free of bile acids and other substances (such as hemoglobin) that can interfere with bilirubin assays. These

reference compositions typically contain a wet or dry matrix and the desired amount of isolated $B_c$. A buffer, e.g., a phosphate buffer, may also be present. In the case of a reference composition for assay of $B_c$ in serum, a reference composition can contain isolated $B_c$ and a serum matrix derived, for example, from pooled human serum or other aqueous medium that, under the conditions of the desired assay, behaves as serum does. Of course, the $B_c$ can be combined with matrices of other aqueous liquids for the assay of liquids other than serum, such as urine, cerebrospinal fluid, and bile. The concentration of the $B_c$ in such reference compositions will vary widely depending on the liquid to be assayed. Concentration ranges for the $B_c$ content of reference compositions useful for the assay of biological liquids can extend from, for example, 0.1 to 40 mg/dl.

The interactive mordants employed in the present method correspond to the mordants for bilirubin described in U.S. Patent 4,069,017. In general, these mordants have multiple binding sites for bilirubin and contain at least one moiety having a hydrophobic organic matrix containing a charge-bearing cationic group. Such mordants can be non-polymeric or polymeric, with especially preferred embodiments of such mordants being represented by homopolymers or copolymers containing as a repeating unit a unit having a charge-bearing cationic group in a hydrophobic organic matrix. Materials having these properties and compositions bind both $B_c$ and $B_u$ and therefore these materials function as mordants for these bilirubin components. The charge-bearing cationic group present in the mordant typically retains its cationic charge in an aqueous environment regardless of pH fluctuation in the aqueous environment. The charge properties of the cationic group in the interactive mordant are therefore insensitive to pH.

Especially preferred polymeric interactive mordants have, in the polymer chain, monomeric units of Formula II below:

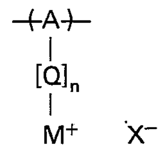

II.

wherein

A represents an organic group and constitutes a portion of a polymer backbone;

n is 0 or 1;

Q represents a group linking $M^+$ to A;

$M^+$ represents a hydrophobic organic moiety containing a cation, preferably a quaternary ammonium or phosphonium group; and

$X^-$ represents an acid anion, for example a halide ion, such as chloride or bromide; nitrate; methosulfate; or p-toluenesulfonate.

In certain especially useful embodiments, $M^+$ represents a quaternary ammonium or phosphonium group having Formulas III or IV below:

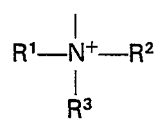

III.

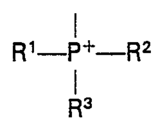

IV.

wherein

each of $R^1$, $R^2$, and $R^3$, which may be the same or different, represents an aryl, an aralkyl, or an alkaryl group preferably having from 5 to 20 carbon atoms or an alkyl group preferably having from 1 to 10 carbon atoms, especially 4 to 10 carbon atoms.

Preferably, Q, in Formula II represents a hydrocarbon group, preferably an arylene, arylene-alkylene, alkylenearylene, and arylenebisalkylene or alkylenebisarylene group. Preferably, Q contains from 5 to 10 carbon atoms.

As will be appreciated, A in Formula II above will vary depending upon the particular polymeric backbone selected for use. Especially good results, however, have been obtained when A represents an alkylene group. Typically, such alkylene groups contain from 2 to 10 carbon atoms.

Copolymers particularly useful as interactive mordants include copolymers containing recurring units having Formula II hereinabove, and, in addition, up to 75 weight percent of additional non-interfering repeating units. The term "non-interfering repeating units" is used herein to include units which do not chemically or physically interfere with the above-described mordanting of bilirubin. Monomers which provide such non-interfering repeating units and which also impart hydrophobicity to

the resultant mordant copolymer include aliphatic and aromatic hydrocarbons, such as olefins and substituted olefins and styrene and substituted styrenes; alkylacrylates and methacrylates and derivatives thereof; and known equivalents for such monomers. In addition, if desired, difunctional crosslinking groups can be introduced into such copolymers.

The preferred polymeric interactive mordants described above have been found to exhibit even greater binding affinity for bilirubin than endogenous serum proteins such as albumin and ligandin.

The preferred polymeric interactive mordants used in the present method are only partially soluble in water. Therefore, these mordants can readily be separated from aqueous mixtures thereof by various means, such as centrifuging or filtration.

A partial listing of individual representative interactive mordants useful in the method of the invention include the following materials. In the copolymers, the weight ratio of the two monomers entering into the polymerization reaction is 50:50, except for polymer 6, where it is 49.5:49.5:1.

TABLE I

| | Name | Structure |
| --- | --- | --- |
| 1. | Poly(N,N,N-trimethyl-N-vinyl-benzylammonium chloride) | 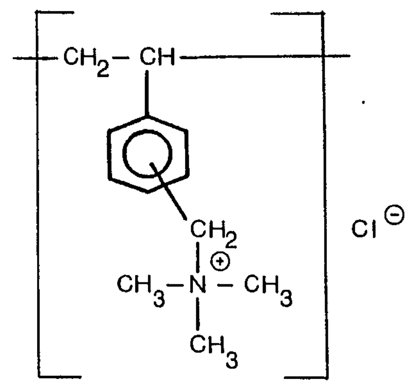 |
| 2. | Poly[styrene-co-benzyl-(dimethyl)-p-vinyl-benzylammonium chloride] | 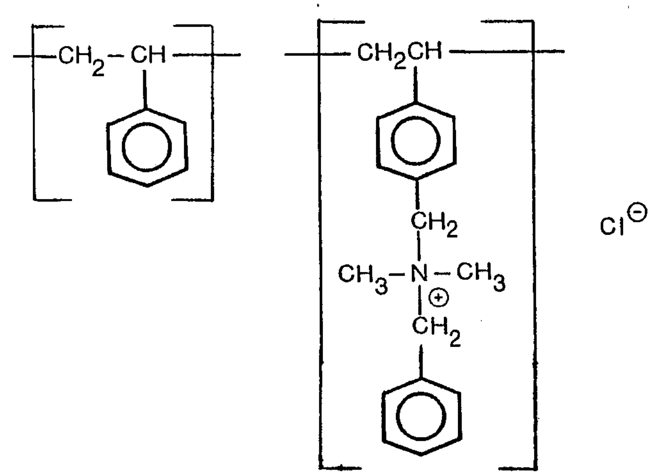 |

TABLE I (Continued)

| | Name | Structure |
|---|---|---|
| 3. | Poly(N,N,N-trioctyl-N-vinyl-benzylphosphonium chloride | 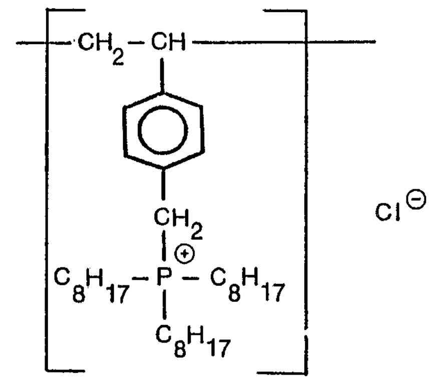 |
| 4. | Poly[styrene-co-(vinylbenzyl)-(trihexyl)-ammonium chloride] | 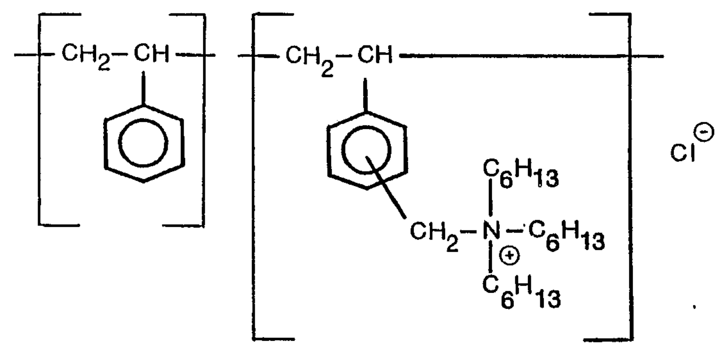 |

TABLE I (Continued)

| | Name | Structure |
|---|---|---|
| 5. | Poly(N,N,N-trimethyl-N-vinyl-benzylammonium chloride-co-styrene) | 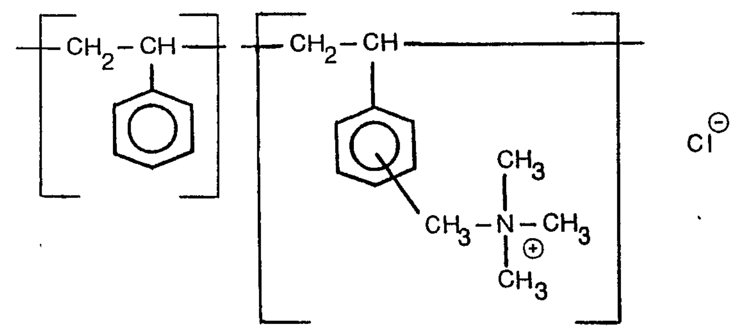 |
| 6. | Poly(styrene-co-N-vinyl-benzyl-N-benzyl-N,N-dimethylammonium chloride-co-divinylbenzene) | 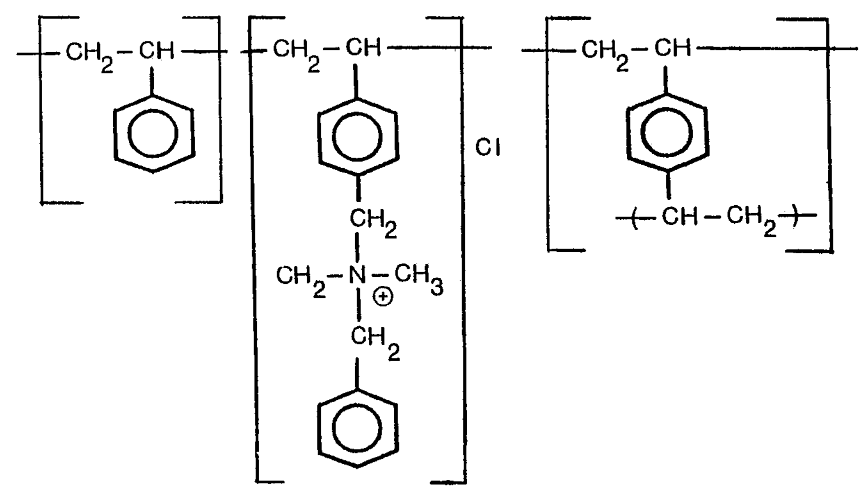 |

Further description of such interactive mordants and methods of preparing them can be found in U.S. Patent 4,069,017.

The following examples are presented to illustrate further the invention. The materials and procedures noted below were used in the examples:

*Chemicals*

Unless otherwise specified, all chemicals used were reagent grade. Sodium thiosulfate, $Na_2S_2O_3$ and the dihydrate of oxalic acid were purchased from Matheson, Coleman and Bell Manufacturing Chemists, Norwood, Ohio 45212. The polymeric mordants noted as mordants 4 and 6 refer to the mordants of the same number listed in Table I. All other chemicals in the examples including unconjugated bilirubin (except where it is specially referred to as being isolated from an aqueous liquid) were obtained from Eastman Organic Chemicals, Eastman Kodak Co., Rochester, New York, 14650.

*Film Element*

The dry test element referred to as a "film" or "bilirubin film" in Example 1 represents an integral multilayer bilirubin test element as described in U.S. Patent 4,069,017. The element is composed of a surface spreading layer for distributing an aqueous sample to an underlying reagent layer containing mordant 6 of Table I which, in turn, is coated on a transparent poly(ethylene terephthalate) film base. The structure and composition of this "film" is similar to that described in detail in Example 2 of U.S. Patent 4,069,017, except that the reagent layer also contained 8.608 g/m$^2$ of deionized gelatin and 0.2M bicine buffer to provide a layer having a pH of 8.0 when spotted with 10 microliters of serum and the spreading layer also contained 2.69 g/m$^2$ caffeine and 4.035 g/m$^2$ sodium benzoate.

*Serum Samples*

Sera from jaundiced adults were specially supplied by local hospitals. All contained, according to versions of the Jendrassik-Grof assay, (as described in With, T. K. in *Bile Pigments, Chemical, Biological And Clinical Aspects;* Academic Press, New York and London, pp 360—410, 1968) a minimum of 10 mg/dl total bilirubin ($B_T$), of which 50—80% appeared as "direct-reacting bilirubin", sometimes considered to represent a measure of $B_c$. The freshly delivered samples were kept frozen at —25°C and in the dark for less than a week, or were processed immediately upon arrival.

*Centrifuging*

All centrifuging in the examples were conducted at 0—4°C in a Beckman JC—21 centrifuge (Beckman Instrument Co.), using a JA—20 rotor.

Example 1 — Film-Based Method

Serum pooled from 3—4 jaundiced-patient samples (each about 1—2 ml) was kept in the dark, on ice, and under a steady stream of nitrogen. A disposable pipette was used to apply about 0.5—1 ml of the serum pool in broad streaks (with total area of 7.5—12 cm$^2$) over a sheet of bilirubin film as described above. As soon as the last trace of fluid had penetrated the surface of the spreading layer (where application was made), another application was repeated over the same area. This was repeated until the serum pool was exhausted. The wetting was conducted in subdued light and under a nitrogen stream. When the wetting was completed, the spreading layer was gently, but thoroughly, removed (this can be done with the edge of a microscope slide). The spreading layer was discarded. Next, the exposed mordant-containing reagent layer, which appeared yellowish, was similarly separated from the film base and collected into thick-walled 50-ml centrifuge tubes. 1.0 ml of n-propanol and 1.0 ml of 2M freshly prepared sodium chloride were added consecutively to each tube. The tube was sealed under nitrogen, shaken vigorously for 30 seconds, and left to stand at room temperature for 1—2 hours in the dark. The solution was then centrifuged for 10 minutes at 15,000 rpm. The yellowish colored supernatant was gently pipetted off, leaving a faintly yellow-colored pellet. The pellet was washed with propanol-NaCl twice as before and the washings were combined with the supernatant. The supernatant still contained small amounts of proteins and mordant. 2 ml of a caffeine-sodium benzoate solution (0.1M:0.2M, final concentrations) were added to the supernatant under nitrogen and the mixture was stirred for one minute. Then the solution was adjusted to pH 5.5 with 1% oxalic acid, whereupon the residual protein and $B_u$ were precipitated, leaving $B_c$ in solution. The acidified $B_c$ solution was centrifuged at 10,000 rpm for 10 minutes. The pH of the supernatant was then readjusted to pH 7.0 with NaOH, mixed with an equal volume of ice-chilled chloroform under nitrogen and shaken for 30 seconds. The whole mixture was centrifuged at 5,000 x g for 15 minutes and the aqueous supernatant was pipetted off carefully. For some preparations, this was the final step and the aqueous extract containing $B_c$ was freeze-dried overnight. Other preparations were further purified by passing the aqueous extract through a column packed with LH—20 gel beads (Pharmacia, Uppsala, Sweden) and 95% ethanol diluted 1:1 (v/v) with 0.1M potassium phosphate buffer, pH 7.0. The same solvent mixture served as the eluting medium. The eluted yellow fraction was immediately dried, first under nitrogen, then under vacuum to give a dark brownish powder of concentrated $B_c$. Further results from this Example are summarized in Example 3.

Example 2 — Solution-Based Method

The following method was found to be particularly suitable for extracting $B_c$ from jaundiced sera having a total bilirubin concentration equal to or in excess of 20 mg/dl, of which 70—80% appeared as "direct" bilirubin in a Jendrassik-Grof assay. Several of these samples appeared lipemic as well.

First, a pool of serum having a high $B_c$ concentration was diluted 1 to 5 (v/v) with distilled water under nitrogen. This solution was carefully titrated with mordant 4 (made up to 1% fresh in water containing 5—10% methanol) until the absorbance at 425 nm stopped increasing. With a mild excess of mordant 4, the absorbance at 425 nm decreased slightly. The titrated solution appeared turbid and was rapidly centrifuged for 15 minutes at 15,000 g at 0—4°C. The yellowish mordanted pellet was gently resuspended in 3 volumes of 0.05 to 0.1M potassium phosphate buffer, pH 7.0, to which 1% $Na_2S_2O_3$ had been added. A fresh solution of caffeine and sodium benzoate was added dropwise to the cloudly suspension until the final level of caffeine was approximately 0.1M and that of benzoate 0.2M. During this time, the solution was stirred vigorously under nitrogen and in a bath of ice. The solution was allowed to stand for $\frac{1}{2}$ hour in the dark and cold (0—4°C), then centrifuged at 10,000 x g for 15 minutes. The supernatant was saved. The pellet was washed at least twice with an equal volume of 0.1M potassium phosphate buffer, pH 7.0, then three to four times with a 1:1 (v/v) mixture of 1M NaCl and n-propanol. The washings were pooled with the supernatant from the preceding centrifugation, then stirred under nitrogen and in the dark for $\frac{1}{2}$ hour. The solution was freeze-dried; the resultant powder was resuspended in a minimal amount of water and loaded on a column packed with LH—20 gel beads and 95% ethanol diluted 1:1 (v/v) with 0.1M potassium phosphate buffer, pH 7.0. The same solvent mixture served as the eluting medium. The yellow eluted fractions were pooled, rechromatographed on a fresh column as before, and immediately freeze-dried. The resulting brownish-yellow powder contained concentrated $B_c$ having a purity of about 85% by weight based on quantitative NMR and gravimetric analysis. Further results from this Example are summarized in Example 3.

Example 3 — Spectroscopic Analysis And Summary of Results

A direct spectroscopic analysis of the $B_c$-containing powder obtained in both Examples 1 and 2 above was made by use of quantitative nuclear magnetic resonance and desorption mass spectrometry. The results of these analyses appear to confirm that the powder obtained in each example contained $B_c$ having the structure noted as Formula I above. In addition, the serum-isolated $B_c$ obtained by the procedures of Examples 1 and 2 was compared to that isolated from bile using the Lucassen procedure. The serum-isolated $B_c$ obtained by the procedures of Examples 1 and 2 was found to be very similar to that from bile in terms of physical appearance, solubility, hygroscopicity, lability to air and light, diazo reactivity, and chromatographic behavior.

The powdered $B_c$-containing isolates prepared by either Example 1 or 2 were kept in the dark, at −25°C, under vacuum and in a desiccated environment. Under these conditions of keeping, there was no discernible change in the visible spectral characteristics of the powdered $B_c$-containing isolates for periods up to two months. If exposed to air, moisture, or light, the powder readily turned green. When the $B_c$-containing powder was reconstituted with water in the presence of 2—4 g% human serum albumin, the $B_c$ appeared significantly more stable.

Further results from the procedures of Examples 1 and 2 to isolate $B_c$ are summarized in Table II. The data illustrate that regardless of the method, the nominal yields (expressed as mg "dry" weight per 10 ml of pooled serum) were highly comparable. The yields thus obtained appeared substantially higher than those reported to date in With, T. K., *Bile Pigments, Chemical, Biological and Clinical Aspects,* Academic Press, New York and London, 1968. This is attributed to the use of the mordants described herein which appear to be highly specific for $B_c$ and $B_u$ and to stabilize the otherwise highly labile $B_c$ during the isolation procedure.

TABLE II
Summary of Serum $B_c$ Isolation

| Isolation Procedure | Example 1 — Film-Based Method | Example 2 — Solution-Based Method |
|---|---|---|
| Starting volume (ml) | 5—8 ml (range of 3 separate pools) | 5—10 ml (range of 2 separate pools) |
| Type of sera pooled | Mostly freshly drawn, with pH range 7.5—7.9 $B_T$ 20 mg/dl and nominal $B_c$ 50% $B_T$ | Mostly freshly delivered, some frozen (—25°C) for a few days before processing; $B_T$ 20 mg/dl with 50% $B_c$, pH range 7.38—8.15 included highly lipemic sera |
| Estimated total mg "$B_c$" in starting pools | 2.2—3.0 | 1.8—3.5 |
| Yield (mg) — apparent dry weight** of isolate per ml of serum pool | 0.83—0.98 (with LH—20 column step) 0.95—1.16 (without LH—20 step) | 0.42—1.55 |
| General physical characteristics | Fluffy dark-brownish powder; hygroscopic; very water-soluble; turns green in moist air or in light | |
| Diazo reactivity | Reacts instantaneously and positively with the diazo reagent,*** giving purplish-pink product even in absence of promotor (or alcohol) | |
| Thin-Layer Chromatography (TLC) | Remains near origin of TLC plates (composed of silica gel G from Eastman Organic Chemicals when run in $CHCl_3$:$CH_3OH$:$H_2O$ (95:30:4, v/v) while authentic $B_u$ (Eastman Organic Chemicals) runs with distinctly higher mobility; mobility of serum $B_c$ isolate similar to bile $B_c$ isolate | |
| Chromatographic behavior | Gel filtration on LH—20 column packed in 95% ethanol | Elution position indicates molecular mass in the range of 900—1000 when judged against known molecular markers. However, the presence of even small amounts of protein (e.g., 0.5 g% human serum albumin) has been observed to alter significantly elution volume of the major fraction in serum isolates |

**Since $B_c$ is very hygroscopic, this dry weight may be subject to some error.
***Manual Jendrassik-Grof assay as described in Routh, J. I., *Fundamentals of Clinical Chemistry,* N. W. Tietz, Ed, W. B. Saunders Co., Philadelphia, London, Toronto (1970) pp 743—762.

Example 4 — Extraction of $B_c$ and $B_u$ From Aqueous Liquids of Non-Human Origin

The method of the present invention can be used to separate both bilirubin components, i.e., $B_c$ and/or $B_u$, from aqueous mammalian liquids other than those of human origin. In this example, a modified "Solution-Based Method" similar to that of Example 2 above was employed to successfully separate $B_c$ and $B_u$ from both dog and rabbit bile. In this example, the procedure of Example 2 was modified as follows: Mordant No. 6 of Table I was used in place of Mordant No. 4 of Table I. The $B_c$ and $B_u$ extracted by the mordant were released from the mordant by washing the mordanted $B_c$ and $B_u$ with a 1% aqueous solution of the non-ionic surfactant octyl phenoxypolyethoxy ethanol, Triton X—100, a tradename of Rohm & Haas Co., having a pH of about 7—7.4, followed by washing with an aqueous caffeine-sodium benzoate solution having a pH of about 7.0 and containing a concentration of caffeine and sodium benzoate of 0.1 M and 0.2 M, respectively. The resultant washings were pooled and contained both $B_c$ and $B_u$ released from the mordant. The mixture of $B_c$ and $B_u$ thus obtained, i.e., the total bilirubin, was then further purified by passing the total bilirubin in a liquid medium composed of a chloroform-methanol-water mixture (95:35:6 v/v) through a column packed with LH—20 gel beads. $B_u$ and $B_c$ were then separately removed from the column. $B_u$ was removed by using dichloromethane as the eluting medium. Thereafter, $B_c$ was removed from the column using distilled water as the eluting medium. The two purified bilirubin components thus obtained were pooled and dried under nitrogen. The yield of total bilirubin obtained by this procedure, average over several repeats of the procedure on

rabbit bile, ranged from 50—80%. The yield of total bilirubin obtained by this procedure, averaged over several repeats of the procedure on dog bile, ranged from 30 to 50%.

**Claims**

1. A method for the separation and isolation of conjugated bilirubin ($B_c$) and unconjugated bilirubin ($B_u$) from an aqueous liquid containing $B_c$ and/or $B_u$, said method characterized by:

a) bringing the liquid and a interactive mordant for $B_c$ and $B_u$ into contact;

b) separating the $B_c$ and/or $B_u$ mordanted in step (a) from the liquid; and

c) treating the separated mordanted $B_c$ and/or $B_u$ of step (b) in an aqueous medium with a chaotropic agent which is soluble in the medium to release at least a portion of the $B_c$ and/or $B_u$ from the mordant.

2. The method of claims 1 wherein the chaotropic agent is selected from ionizable salts, nonionic surfactants, xanthine, alkylated xanthines, urea or mixtures thereof.

3. The method of claims 1 or 2 wherein the mordant is a polymeric mordant having units in the chain of the formula

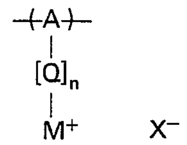

II

wherein

A represents an organic group constituting a portion of the polymer backbone;

n is 0 or 1;

Q represents a group linking $M^+$ to A;

$M^+$ represents a hydrophobic organic moiety containing a cation; and

$X^-$ represents an acid anion.

4. The method of claim 3 wherein the polymeric mordant is a copolymer containing 10—90 wt.% of repeating units having formula II as defined in claim 3 and up to 75 wt.% of repeating units of a non-interfering hydrophobic monomer.

5. The method of any of claims 1—4 wherein the $B_u$ and $B_c$ are present in the aqueous liquid as a mixture and wherein the method is further characterized by step (d): selectively dissolving the released $B_c$ or $B_u$ of step (c) to separate $B_c$ from $B_u$.

6. The method of claim 5 wherein step (d) is carried out during or after step (c) by adjusting the pH of the aqueous medium to a value at which the released $B_c$ is soluble therein, while the released $B_u$ forms an insoluble solid phase.

7. The method of claim 6 wherein the adjusted pH is less than 7.0.

8. The method of claim 5 wherein step (d) is carried out by extraction in a water-immiscible organic solvent to dissolve $B_u$.

9. The method of claim 8 wherein the water-immiscible organic solvent is chloroform, dichloromethane, or a mixture thereof.

10. The method of any of claims 1—7 wherein a volatile water-miscible organic solvent which is a solvent for $B_c$ but not for $B_u$ is added to the mordanted $B_c$ and/or $B_u$ following step (b).

11. The method of claim 10 wherein the volatile water-miscible organic solvent is added to the mordanted $B_c$ and/or $B_u$ in step (c).

12. The method of claims 10 or 11 wherein the volatile, water-miscible organic solvent is propanol.

13. The method of any of claims 1—12 wherein the chaotropic agent is the salt of a strong acid and a strong base.

14. The method of any of claims 1—13 wherein the method is carried out at a temperature in the range of from 0 to 60°C.

15. The method of any of claims 1—14 wherein the method is carried out in a non-oxidizing atmosphere.

16. The method of any of claims 1—15 wherein step (a) is carried out in a dry test element.

17. The method of any of claims 1—15 wherein step (a) is carried out by bringing the interactive mordant and the aqueous liquid into contact in an aqueous medium.

18. The method of any of claims 1—17 wherein the method is carried out at a temperature in the range of from 0 to 10°C.

19. Isolated conjugated bilirubin characterized by a purality in excess of 75% by weight.

20. A reference composition for the assay of bilirubin contained in an aqueous liquid, the composition including a wet or dry matrix and the isolated conjugated bilirubin of claim 19.

## Patentansprüche

1. Verfahren zur Trennung und Isolierung von konjugiertem Bilirubin ($B_c$) und unkonjugiertem Bilirubin ($B_u$) aus einer wäßrigen Flüssigkeit mit einem Gehalt an $B_c$ und/oder $B_u$, dadurch gekennzeichnet, daß man:

a) die Flüssigkeit mit einem reaktionsfähigen Beizmittel für $B_c$ und $B_u$ in Kontakt bringt;

b) das in der Stufe (a) gebeizte $B_c$ und/oder $B_u$ von der Flüssigkeit abtrennt; und

c) das abgetrennte gebeizte $B_c$ und/oder $B_u$ von Stufe (b) in einem wäßrigen Medium mit einem chaotropen Mittel behandelt, das in dem Medium löslich ist, unter Freisetzung mindestens eines Teiles des $B_c$ und/oder $B_u$ von dem Beizmittel.

2. Verfahren nach Anspruch 1, dadurch gekennzeichnet, daß man das chaotrope Mittel aus ionisierbaren Salzen, nicht-ionogenen oberflächenaktiven Stoffen, Xanthin, alkylierten Xanthinnen, Harnstoff oder Mischungen hiervon auswählt.

3. Verfahren nach Anspruch 1 oder 2, dadurch gekennzeichnet, daß das Beizmittel ein polymeres Beizmittel mit Einheiten in der Kette der Formel:

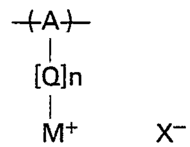

II

ist, worin bedeuten:

A eine organische Gruppe, die einen Teil der Polymerkette bildet;

n = 0 oder 1;

Q eine Gruppe, die $M^+$ mit A verbindet;

$M^+$ einen hydrophoben organischen Rest mit einem Kation und

$X^-$ eine Säureanion.

4. Verfahren nach Anspruch 3, dadurch gekennzeichnet, daß das polymere Beizmittel ein Copolymer mit 10—90 Gew.-% wiederkehrenden Einheiten der Formel II, wie in Anspruch 3 definiert, und mit bis zu 75 Gew.-% wiederkehrenden Einheiten aus einem nichtstörenden hydrophoben Monomer ist.

5. Verfahren nach einem der Ansprüche 1 bis 4, dadurch gekennzeichnet, daß $B_u$ und $B_c$ in der wäßrigen Flüssigkeit in Form einer Mischung vorliegen und daß das Verfahren weiter gekennzeichnet ist durch die Stufe (d): wonach das freigesetzte $B_c$ oder $B_u$ von Stufe (c) zur Trennung von $B_c$ von $B_u$ selektiv gelöst wird.

6. Verfahren nach Anspruch 5, dadurch gekennzeichnet, daß die Stufe (d) während oder nach der Stufe (c) durchgeführt wird durch Einstellung des pH-Wertes des wäßrigen Mediums auf einen Wert, bei dem das freigesetzte $B_c$ in dem Medium löslich ist, während das freigesetzte $B_u$ eine unlösliche feste Phase bildet.

7. Verfahren nach Anspruch 6, dadurch gekennzeichnet, daß der eingestellte pH-Werte bei weniger als 7,0 liegt.

8. Verfahren nach Anspruch 5, dadurch gekennzeichnet, daß die Stufe (d) durch Extraktion in einem mit Wasser nicht mischbaren organischen Lösungsmittel zur Lösung von $B_u$ durchgeführt wird.

9. Verfahren nach Anspruch 8, dadurch gekennzeichnet, daß das mit Wasser nicht mischbare organische Lösungsmittel aus Chloroform, Dichlormethan oder einer Mischung hiervon besteht.

10. Verfahren nach einem der Ansprüche 1 bis 7, dadurch gekennzeichnet, daß ein flüchtiges, mit Wasser mischbares organisches Lösungsmittel, welches ein Lösungsmittel für $B_c$, jedoch nicht für $B_u$ ist, zu dem gebeizten $B_c$ und/oder $B_u$ nach der Stufe (b) zugesetzt wird.

11. Verfahren nach Anspruch 10, dadurch gekennzeichnet, daß man das flüchtige, mit Wasser mischbare organische Lösungsmittel zu dem gebeizten $B_c$ und/oder $B_u$ in Stufe (c) zugibt.

12. Verfahren nach Anspruch 10 oder 11, dadurch gekennzeichnet, daß man als flüchtiges, mit Wasser mischbares organisches Lösungsmittel Propanol verwendet.

13. Verfahren nach einem der Ansprüche 1 bis 12, dadurch gekennzeichnet, daß das chaotrope Mittel das Salz einer starken Säure und einer starken Base ist.

14. Verfahren nach einem der Ansprüche 1 bis 13, dadurch gekennzeichnet, daß das Verfahren bei einer Temperatur von 0 bis 60°C durchgeführt wird.

15. Verfahren nach einem der Ansprüche 1 bis 14, dadurch gekennzeichnet, daß das Verfahren in einer nicht-oxydierenden Atmosphäre durchgeführt wird.

16. Verfahren nach einem der Ansprüche 1 bis 15, dadurch gekennzeichnet, daß die Stufe (a) in einem trockenen Testelement durchgeführt wird.

17. Verfahren nach einem der Ansprüche 1 bis 15, dadurch gekennzeichnet, daß die Stufe (a) dadurch durchgeführt wird, daß man das reaktionsfähige Beizmittel und die wäßrige Flüssigkeit in einem wäßrigen Medium miteinander in Kontakt bringt.

18. Verfahren nach einem der Ansprüche 1 bis 17, dadurch gekennzeichnet, daß man das Verfahren bei einer Temperatur von 0 bis 10°C durchführt.

19. Isoliertes konjugiertes Bilirubin, gekennzeichnet durch eine Reinheit von über 75 Gew.-%.

20. Vergleichsmaterial für die Bestimmung von Bilirubin in einer wäßrigen Flüssigkeit, enthaltend eine feuchte oder trockene Matrix und das isolierte, konjugierte Bilirubin von Anspruch 19.

**Revendications**

1. Procédé pour séparer et isoler la bilirubine conjuguée (Bc) et la bilirubine non conjuguée (Bnc) à partir d'un liquide aqueux contenant Bc et/ou Bnc, le dit procédé étant caractérisé en ce que:

(a) on amène en contact le liquide et un mordant capable de réagir avec Bc et Bnc;

(b) on sépare du liquide les composés Bc et/ou Bnc mordancés dans l'étape (a); et

(c) on traite les composés mordancés séparés Bc et/ou Bnc, dans un milieu aqueux, avec un agent chaotrope qui est soluble dans le milieu, de façon à libérer du mordant au moins une partie de Bc et/ou Bnc.

2. Procédé conforme à la revendication 1, dans lequel l'agent chaotrope est choisi dans le groupe constitué par les sels ionisables, les agents tensioactifs non ioniques, la xanthine, les xanthines alkylées, l'urée ou des mélanges de ces composés.

3. Procédé conforme aux revendications 1 ou 2, dans lequel le mordant est un mordant polymère comprenant dans la chaîne des motifs de formule:

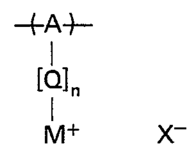

II

dans laquelle

A représente un groupe organique constituant une partie du squelette du polymère;

n est égal à 0 ou à 1;

Q représente un groupe reliant $M^+$ à A;

$M^+$ représente un motif organique hydrophobe contenant un cation; et

$X^-$ représente un anion acide.

4. Procédé conforme à la revendication 3, dans lequel le mordant polymère est un copolymère contenant de 10% à 90% en masse de motifs ayant la formule II telle que définie à la revendication 3 et jusqu'à 75% en masse de motifs d'un monomère hydrophobe non interférant.

5. Procédé conforme à l'une quelconque des revendications 1 à 4, dans lequel les composés Bnc et Bc sont présents sous forme de mélange dans le liquide aqueux et dans lequel le procédé est en outre caractérisé par l'étape (d) consistant à dissoudre sélectivement les composés Bc et Bnc libérés dans l'étape (c) pour séparer Bc de Bnc.

6. Procédé conforme à la revendication 5, dans lequel on effectue l'étape (d) pendant ou après l'étape (c), en ajustant le pH du milieu aqueux à une valeur à laquelle le composé libéré Bc est soluble dans le dit milieu tandis que le composé Bnc libéré forme une phase solide insoluble.

7. Procédé conforme à la revendication 6, dans lequel on ajuste le pH à une valeur inférieure à 7,0.

8. Procédé conforme à la revendication 5, dans lequel on effectue l'étape (d) par extraction dans un solvant organique non miscible à l'eau pour dissoudre Bnc.

9. Procédé conforme à la revendication 8, dans lequel le solvant organique non miscible à l'eau est le chloroforme, le dichlorométhane ou un mélange de ces composés.

10. Procédé conforme à l'une quelconque des revendications 1 à 7, dans lequel on ajoute aux composés mordancés Bc et/ou Bnc, après l'étape (b), un solvant organique volatil miscible à l'eau, qui est un solvant de Bc mais non de Bnc.

11. Procédé conforme à la revendication 10, dans lequel on ajoute aux composés mordancés Bc et/ou Bnc, au cours de l'étape (c), le solvant organique volatil miscible à l'eau.

12. Procédé conforme aux revendications 10 et 11, dans lequel le solvant organique volatil miscible à l'eau est le propanol.

13. Procédé conforme à l'une quelconque des revendications 1 à 12, dans lequel l'agent chaotrope est le sel d'un acide forte et d'une base forte.

14. Procédé conforme à l'une quelconque des revendications 1 à 13, dans lequel on met en oeuvre le procédé à une température comprise entre 0°C et 60°C.

15. Procédé conforme à l'une quelconque des revendications 1 à 14, dans lequel on met en oeuvre le procédé en atmosphère non oxydante.

16. Procédé conforme à l'une quelconque des revendications 1 à 15, dans lequel on effectue l'étape (a) dans un élément d'essai par voie sèche.

17. Procédé conforme à l'une quelconque des revendications 1 à 15, dans lequel on effectue

l'étape (a) en amenant en contact, dans un milieu aqueux, le mordant et le liquide aqueux.

18. Procédé conforme à l'une quelconque des revendications 1 à 17, dans lequel on met en oeuvre le procédé à une température comprise entre 0°C et 10°C.

19. Bilirubine conjuguée isolée, caractérisée par une pureté excédant 75% en masse.

20. Composition de référence pour l'essai de la bilirubine contenue dans un liquide aqueux, la composition comprenant une matrice humide ou sèche et la bilirubine conjuguée isolée de la revendication 19.